(19) Europäisches Patentamt
European Patent Office
Office européen des brevets

(11) Veröffentlichungsnummer : **0 044 970**
**B1**

(12) # EUROPÄISCHE PATENTSCHRIFT

(45) Veröffentlichungstag der Patentschrift : **22.06.83**

(51) Int. Cl.³ : **A 61 K 7/42**

(21) Anmeldenummer : **81105199.4**

(22) Anmeldetag : **04.07.81**

(54) **Verwendung von verätherten 2-(4-Hydroxybenzyliden)-gamma-butyrolactonen als Lichtschutzmittel.**

(30) Priorität : **26.07.80 DE 3028502**

(43) Veröffentlichungstag der Anmeldung : **03.02.82 Patentblatt 82/05**

(45) Bekanntmachung des Hinweises auf die Patenterteilung : **22.06.83 Patentblatt 83/25**

(84) Benannte Vertragsstaaten : **CH DE FR GB IT LI**

(56) Entgegenhaltungen :
**DD A 112 884**
**DE A 2 160 136**
**Journal of Organic Chemistry, Band 24, Januar 1959 EASTON (US) H. ZIMMER et al. : « Substituted gamma-Lactones. I. Preparation of gamma-Substituted gamma-Butyrolactones by Condensation of gamma-Butyrolactone with Aldehydes. Hydrogenation of the Condensation Products » Seiten 28-32**

(73) Patentinhaber : **BASF Aktiengesellschaft**
**Carl-Bosch-Strasse 38**
**D-6700 Ludwigshafen (DE)**

(72) Erfinder : **Thoemel, Frank, Dr.**
**Leberstrasse 17**
**D-6940 Weinheim (DE)**
Erfinder : **Hoffmann, Werner, Dr.**
**Ringstrasse 11 C**
**D-6701 Neuhofen (DE)**
Erfinder : **Degner, Dieter, Dr.**
**Kurpfalzstrasse 8**
**D-6701 Dannstadt-Schauernheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 044 970 B1

Jouve, 18, rue St-Denis, 75001 Paris, France

# Verwendung von verätherten 2-(4-Hydroxybenzyliden)-γ-butyrolactonen als Lichtschutzmittel

Die Erfindung betrifft die Verwendung von an der phenolischen Hydroxylgruppe veretherten 2-(4-Hydroxybenzyliden)-γ-butyrolactonen als Lichtschutzmittel und UV-Stabilisatoren insbesondere für die menschliche Haut.

Es ist bekannt, daß das 2-(4-Methoxybenzyliden)-γ-butyrolacton gemäß der DDR-PS 112 884 als Pflanzenwuchsregulator verwendet werden kann.

Aus J. Org. Chem. 24 (1959) Seite 30 sind weitere derartige Butyrolactone bekannt, die als Zwischenprodukte für daraus erhältliche hydrierte Lactone vorgeschlagen werden, die hinwiederum auf dem pharmazeutischen Sektor interessant sein sollen.

Weiterhin ist bekannt, daß der als UV-B-Strahlung bezeichnete Bereich zwischen 280 und 315 nm des Sonnenlichts oder künstlicher Lichtquellen für die Erythembildung der menschlichen Haut verantwortlich ist. Das Maximum der Wirksamkeit der UV-Strahlung für die Erythembildung liegt bei 297 nm, wenn die Strahlungsintensität für alle Wellenlängen gleich groß ist. Beim Sonnenlicht mit Strahlung unterschiedlicher Intensität ist dieses Maximum auf 308 nm verschoben. Durch geeignete Filtersubstanzen für den UV-B-Bereich gelingt es, die Erythembildung zu verhindern oder zumindest zu verzögern. Die Pigmentbildung der Haut, d.h. die Bräunung, soll dagegen gewährleistet bleiben.

Die UV-Strahlung ist darüber hinaus auch eine wichtige Einflußgröße bei der Alterung von Polymeren und kann beispielsweise die Umwandlung bestimmter Farbstoffe bewirken, so daß auch für solche Produkte Filtersubstanzen als Stabilisatoren nahezu unentbehrlich sind.

In den vergangenen vierzig Jahren ist eine große Anzahl von chemischen Verbindungen auf ihre Filterwirkung im UV-B-Bereich hin untersucht worden. Ob es sich jedoch bei einer im UV-Gebiet absorbierenden Substanz auch für die menschliche Haut um einen brauchbaren Sonnenschutzfilter handelt, wird noch von anderen Faktoren entscheidend bestimmt:

Neben der hohen Filterwirksamkeit in dem erythemalen Bereich soll die Substanz eine hohe Durchlässigkeit für die Bräunungsenergie aufweisen, sie sollte eine möglichst ideale Haut- und Schleimhautverträglichkeit besitzen und darf nicht toxisch sein. Sie darf nicht oxidationsempfindlich sein und durch UV-Bestrahlung keine Veränderungen oder Verfärbungen erleiden. Eine entsprechende Zubereitung soll lagerstabil sein, keinen Eigengeruch aufweisen und mit den üblicherweise verwendeten Trägerstoffen oder Verdünnungsmitteln verträglich sein.

Die bekannten UV-Filter weisen häufig als Nachteile auf, daß sie beim Lagern sowie gegen UV-Strahlung oder sichtbarer Strahlung und/oder Luft instabil sind, in gefärbte Zersetzungsprodukte übergehen, die Wäsche anschmutzen oder sogar hautschädlich sein können. Da Sonnenschutzmittel häufig von Personen verwendet werden, die im Freien arbeiten, Sport treiben, wie Schwimmen und Tauchen, sollen diese Mittel nicht zu leicht durch Wasser oder Schweiß von der Haut entfernbar sein.

Es gibt nur verhältnismäßig wenig Substanzen, die den angegebenen Ansprüchen mehr oder weniger gut entsprechen. Beispielsweise kann der Literatur, Fette, Seifen, Anstrichmittel, 71. Jahrgang, Seiten 46-48 (1969) oder der DT-OS 2 032 914 entnommen werden, daß von vielen untersuchten Substanzen Ester der p-Aminobenzoesäure oder Ester der p-Dimethylaminobenzoesäure mit Benzoephenonderivaten eine brauchbare Lichtschutzwirkung zeigen.

Es wurde nun gefunden, daß Verbindungen der allgemeinen Formel I

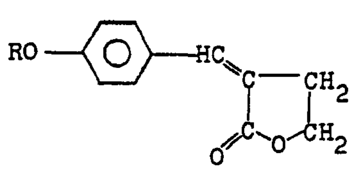

(I)

in der R einen geradkettigen oder verzweigten Alkylrest mit 1 bis 15 Kohlenstoffatomen, einen Phenyl- oder Benzylrest bedeutet, als Lichtschutzmittel für die menschliche Haut in kosmetischen Zubereitungen oder als technisches Lichtschutzmittel oder UV-Stabilisator für Kunststoffe, Lösungs- und Anstrichmittel sowie Öle pflanzlicher oder petrochemischer Herkunft verwendet werden können.

Die Verbindungen der Formel I absorbieren mit sehr guter Extinktion im UV-B-Bereich. Durch Variation der Ätherkomponente kann man Lichtschutzmittel mit gezielten Absorptions- und Löslichkeitseigenschaften erhalten. Die Palette der bisher verfügbaren Lichtschutzmittel wird durch die erfindungsgemäß zu verwendenden und chemisch leicht zugänglichen Verbindungen in vorteilhafter Weise bereichert.

Von den für R angegebenen Bedeutungen sind bevorzugt Alkylreste mit 1 bis 4 Kohlenstoffatomen.

Die erfindungsgemäß zu verwendenden Verbindungen werden in an sich üblicher Weise durch Kondensation eines entsprechend verätherten p-Hydroxybenzaldehyds mit γ-Butyrolacton zweckmäßig in einem Lösungsmittel und in Gegenwart von Alkali hergestellt. Beispielhaft sei die Kondensation in einem aromatischen Kohlenwasserstoff, wie Toluol, bei Temperaturen von 0 bis 100 °C in Gegenwart eines Alkalialkoholats, wie Natriummethylat, angeführt.

Die erfindungsgemäß zu verwendenden Verbindungen finden im Kosmetik-, Farben- und Kunststoffbereich Verwendung. Die neuen Lichtschutzmittel wirken stabilisierend gegenüber Lichteinflüssen in Ölen pflanzlicher und petrochemischer Herkunft sowie Lösungsmittel. Sie können auch im Gemisch mit anderen Lichtschutzmitteln Verwendung finden. Der Gehalt in den Zubereitungen mit den erfindungsgemäß zu verwendenden Verbindungen hängt weitgehend vom Verwendungszweck dieser Zubereitungen ab und liegt im allgemeinen bei 0,1 bis 15 Gewichtsprozent einer Verbindung der allgemeinen Formel I.

Von der Art des Trägers oder Verdünnungsmittels hängt es ab, ob das fertige Lichtschutzmittel beispielsweise eine Lösung, ein Öl, eine Creme, eine Salbe, eine Lotion, eine Emulsion oder ein Pulver ist. Derartige Zubereitungen können beispielsweise der Zeitschrift « Fette und Seifen », 53. Jahrgang, Seiten 694 bis 699 (1951), der Zeitschrift « Seifen, Öle, Fette, Wachse », 1955, Seite 147 oder H. Janistyn, Handbuch der Kosmetika und Riechstoffe, Bd. 3, 1973, Hüthig Verlag, Heidelberg, entnommen werden.

Üblicherweise verwendete kosmetische Hilfsstoffe, die als Zusätze in Betracht kommen, sind beispielsweise Emulgatoren, wie Fettalkoholäthoxylate, Sorbitanfettsäureester oder Lanolinderivate, Dickungsmittel, wie Carboxymethylcellulose oder vernetzte Polyacrylsäure, Konservierungsmittel oder Parfüms.

Grundlage für Sonnenschutzöle sind beispielsweise pflanzliche Öle, wie Erdnußöl, Olivenöl, Sesamöl, Baumwollsamenöl, Kokosöl, Traubenkernöl, Rizinusöl, oder Mineralöle, wie Vaselinöl oder insbesondere flüssiges Paraffin, synthetische Fettsäureester und Glyceride.

Grundlage für Salben sind beispielsweise Vaseline, Lanolin, Eucerin, Polyäthylenglycole oder Fettsäureester.

Grundlage für Cremes sind beispielsweise fettreiche Cremes, Glycerin-, Polysaccarid-, Tylose-Cremes, für Cremes auf Basis von Fetten und Wachsen Cetylalkohol, Lanolincreme, Kakaobutter, Bienenwachs, Stearinsäure, Stearinalkohol, Glycerinmonostearat, native und mineralische Öle und Fette.

Grundlage für Emulsionen sind beispielsweise Mischungen aus Stearinglycol, einem pflanzlichen und/oder Mineralöl, wie Mandelöl, Paraffinöl und Vaseline und Wasser oder Mischungen aus Äthylalkohol, Wasser, Lanolin und Tragant, oder Mischungen aus Äthylalkohol, Stearin, Wasser oder Tragant, Glycerin, Alkohol und Wasser, oder Mischungen aus Stearinsäure, Paraffinöl, Propyl oder Isopropylalkohol und Wasser.

Zur weiteren Erläuterung des Gegenstands der Erfindung sind nachstehend einige Beispiele angeführt.

Beispiel für die chemische Herstellung :

Zu einem Gemisch von 80 g (0,93 mol) γ-Butyrolacton, 64 g (0,47 mol) Anisaldehyd und 200 ml Toluol gibt man unter Rühren in 30 Minuten bei Raumtemperatur portionsweise 35 g (0,64 mol) Natriummethylat und läßt eine Stunde nachrühren. Anschließend stellt man das Reaktionsgemisch mit 10 % Schwefelsäure schwach sauer. Die wäßrige Phase wird abgetrennt und die organische Phase mit 100 ml Äther versetzt, wobei ein kristallines Produkt ausfällt. Das kristalline Produkt wird auf einem Büchnertrichter gesammelt und getrocknet. Man erhält 68 g (71 % d. Th.) Verbindung 1, Tabelle 1. Die Verbindungen 2 bis 6 werden analog hergestellt.

(Siehe die Tabelle 1 Seite 4)

Tabelle 1
Verbindungen der Formel I

| Nr. | Verbindungen | $_{max}$[nm] | (Ethanol) | $E^{1\%}_{1cm}$ (Ethanol | Schm.[$^oC$] |
|---|---|---|---|---|---|
| 1 | 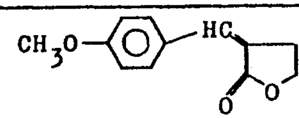 | 309 | 2,58 x $10^4$ | 1263 | 125 - 127 |
| 2 | 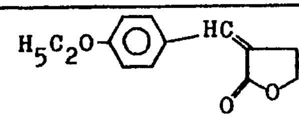 | 311 | 2,50 x $10^4$ | 1238 | 97 - 100 |
| 3 | 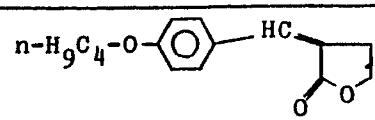 | 310,6 | 2,79 x $10^4$ | 1143 | 94 - 86 |
| 4 | 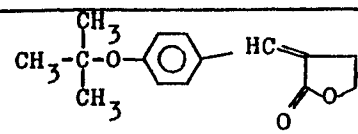 | 297 | 2,51 x $10^4$ | 1018 | 88 - 90 |
| 5 | 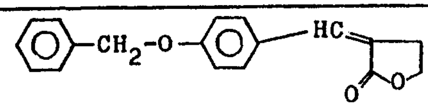 | 310 | 2,65 x $10^4$ | 948 | 162 - 164 |
| 6 | 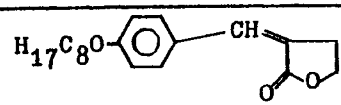 | 311 | 2,47 x $10^4$ | 907 | 75 - 77 |

Beispiele für die Formulierung als Lichtschutzmittel :
Die Zahlen in den nachfolgenden Formulierungsbeispielen sollen Gewichtsteile bedeuten.

Beispiel 1

Sonnenschutzmilch

a) 2,0 Fettalkohol 6-fach ethoxyliert
2,0 Fettalkohol 25-fach ethoxyliert
5,0 2-Äthylhexansäureoctadecylester
4,5 Glycerintristearat
14,0 Paraffinöl
4,0 Stearinsäure
0,5 Cetylalkohol
0,2 p-Hydroxybenzoesäuremethylester
5,5 2-(4-tert.-Butoxybenzyliden)-γ-butyrolacton.
b) 2,0 Triäthanolamin
60,0 Wasser.
c) 0,3 Parfümöl.

Man erwärmt die Phasen a und b auf 70 °C und rührt Phase b in Phase a ein. Wenn das gerührte Gemisch auf 40 °C abgekühlt ist, gibt man Phase c zu und verrührt kurz.

Beispiel 2

Sonnenschutzcreme, weich

a) 1,0 Stearinsäure 9-fach ethoxyliert
1,0 Fettalkohol 6-fach ethoxyliert
1,0 Fettalkohol 25-fach ethoxyliert
10,0 Stearinsäure
2,0 Vaseline
12,0 Paraffinöl
4,4 Bienenwachs hell
0,5 Siliconöl
2,0 2-Äthylhexansäureoctadecylester
4,1 2-(4-Äthoxybenzyliden)-γ-butyrolacton.
b) 4,0 Propylenglykol
0,2 p-Hydroxybenzoesäuremethylester
0,5 Triäthanolamin
57,3 Wasser.

Die Phasen a und b werden auf 75 °C erwärmt und die Phase b in die Phase a eingerührt. Man rührt bis zum Abkühlen auf 40 °C weiter, gibt nach Belieben Parfümöl zu und rührt bis zum Erreichen der Raumtemperatur nach.

Beispiel 3

Sonnenschutzcreme

a) 3,0 Stearinsäure 9-fach ethoxyliert
1,0 Fettalkohol 6-fach ethoxyliert
1,0 Fettalkohol 25-fach ethoxyliert
5,0 Glycerinmonostearat
1,9 Lanolin
1,4 Cetylalkohol
9,0 Stearinsäure
2,0 2-Äthylhexansäureoctadecylester
0,5 Siliconöl
6,2 2-(4-Butoxybenzyliden)-γ-butyrolacton.
b) 0,2 p-Hydroxybenzoesäuremethylester
4,0 Propylenglykol
0,5 Triäthanolamin
64,0 Wasser.
c) 0,3 Parfümöl.

Die Phase a wird auf 80 °C erwärmt. Man rührt dann die auf 70 °C erwärmte Phase b ein. Bei 40 °C gibt man phase d zu und rührt bis zum Erreichen der Raumtemperatur weiter.

**Ansprüche**

1. Verwendung von Verbindungen der Formel I

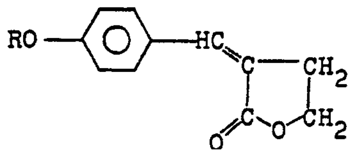

(I)

in der R einen geradkettigen oder verzweigten Alkylrest mit 1 bis 15 Kohlenstoffatomen, einen Phenyl- oder Benzylrest bedeutet, als Lichtschutzmittel oder UV-Stabilisator.

2. Verwendung von Verbindungen gemäß Anspruch 1 als Lichtschutzmittel für die menschliche Haut.

**Claims**

1. The use of a compound of the formula I

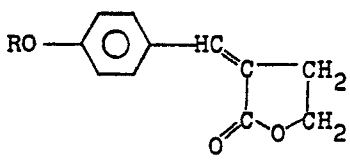

(I)

where R is linear or branched alkyl of 1 to 15 carbon atoms, phenyl or benzyl, as a light stabilizer or ultraviolet absorber.

2. The use of a compound as claimed in claim 1 as a sun screen agent for the human skin.

**Revendications**

1. Utilisation de composés de formule I

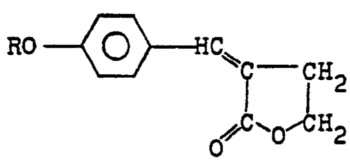

(I)

dans laquelle R représente un reste alkyle à chaîne droite ou ramifiée de 1 à 15 atomes de carbone, un reste phényle ou benzyle, comme agent de protection contre la lumière ou du stabilisateur UV.

2. Utilisation de composés selon la revendication 1, comme agent de protection contre la lumière pour la peau humaine.